# EUROPEAN PATENT SPECIFICATION

(11) **EP 1 490 120 B1**
(45) Date of publication and mention of the grant of the patent: **11.11.2009**
(21) Application number: 03723838.3
(22) Date of filing: 28.03.2003
(51) Int. Cl.: A61L 24/04, A61L 24/00, A61L 27/50, A61L 27/16, A61L 31/04, A61L 31/14, A61K 9/16

(54) **EMBOLIZATION**
EMBOLISATION
EMBOLISATION

(30) Priority: 29.03.2002 US 109966; 04.04.2002 US 116330; 09.08.2002 US 215594
(43) Date of publication of application: 29.12.2004
(73) Proprietor: Boston Scientific Limited, St. Michael (BB)
(72) Inventor: BUISER, Marcia, Watertown, MA 02472 (US); BELLISARIO, Marc, Cambridge, MA 02139 (US); KNAPP, David, Saint Paul, MN 55105 (US); MANGIN, Stephan, Ashland, MA 01721 (US); LANPHERE, Janel, Newton, MA 02461 (US)
(74) Representative: Altenburg, Udo
(86) International application number: PCT/US2003/009408
(87) International publication number: WO 2003/084582

(56) References cited:
- EP-A- 0 402 031
- EP-A- 0 730 847
- WO-A-01/70291
- WO-A-93/19702
- DE-A- 10 026 620
- DE-U- 9 414 868
- US-A1- 2001 051 670
- US-B1- 6 224 630

## Description

This invention relates to embolization and in particular to an embolic composition and a method of manufacture therefor.

### BACKGROUND

Therapeutic vascular occlusions (embolizations) are used to treat pathological conditions *in situ*. Compositions including embolic particles are used for occluding vessels in a variety of medical applications. Delivery of embolic particles through a catheter is dependent on size uniformity and compressibility of the embolic particles.

DE 100 26 620 A discloses a biocompatible material for cell and tissue implantation comprising spherical microspheres with a porous outer cover and one or several inner hollow spaces. The microspheres have a size in the range of 0.01 microns to 100 millimeter. The inner hollow spaces are used for encapsulating simple or complex devices as sensors, microsurgical devices, semiconductor devices, CPUs etc. This biocompatible material is not disclosed as being usable for embolization.

US 2001/051670 A1 discloses an embolic composition comprising macromers forming a hydrogel in the form of a solid microsphere. The macromers have a backbone of a polymer as for example polyvinyl alcohol. The microspheres can be made in sizes ranging from about 10 microns to 2000 microns. The composition can include fillers that leach out of the formed hydrogel over a period of time and cause the hydrogel to become porous. These embolic particles can be delivered through a catheter only with difficulties.

It is therefore the problem of the present invention to provide embolic particles which can be delivered through a catheter without difficulties and to provide a method for manufacturing such particles.

This problem is solved according to the invention by a composition as defined in claim 1 and the method as defined in claim 26. Embodiments of the invention are defined in the dependent claims.

As described later in more detail, the inventive pore size distribution according to the characterizing part of claim 1 enhances on the one side the resistance to shear forces and abrasion due to the small pore size surface region of the particles and on the other side the compressibility due to the large pore size interior region. Both advantages, namely enhancement of the resistance to shear forces and enhancement of the compressibility improve the delivery of the particles through the catheter into the body of a patient. Once in the body, the particles substantially recover to original diameter and shape for efficient transport in the carrier and body fluid stream. At the point of occlusion, the particles can again compress as they aggregate in the occlusion region.

The relatively large pores are 20 or 30 micron or more. The surface region is r to 0.8r. The surface region is r to 2/3r. The particles include a body region from 2/3r to ^{r}/₃ including intermediate size pores and the body region has more intermediate size pores than the surface region. The center region is from ^{r}/₃ to C, the outer region including large size pores and the body region has fewer large size pores than the center region. The intermediate size pores are 2 to 18 microns. The surface region is substantially free of pores greater than 5 micron.

The predominant pore size progressively increases from surface to the center of the particle. The predominant pore size on the particle surface is 1 micron or less. The particles have a surface region from (2r)/3 to the surface wherein the predominant pore size is in the range of 1 micron or less. The predominant pore size is 0.1 micron or less. Interior of said surface region, the particles have a predominant pore size in the range of 2 to 35 microns. The particles include a center region from r to r/3 in which the predominant pore size is 20 to 35 micron. The particles have a body region from r/3 to (2r)/3 in which the predominant pore size is 2 to 18 micron. The particles have a surface region from (2r)/3 to the periphery and the predominant pore size in the surface region is 10% or less than the predominant pore size in the interior to the surface region. The particles include a surface region from 0.8r to r wherein the predominate pore size is 1 micron or less. The particles include a region from C to 0.8r includes pores having a diameter of 10 microns or more. The region C to 0.8r has a predominant pore size of 3.5 to 2 micron. The particles have a density of 1.1 to 1.4 g/cm3. The particles have a density of 1.2 to 1.3 g/cm3. The embolic particles have a sphericity of 90% or more. The particles have an initial sphericity of 97% or more. The particles have a sphericity of 0.90 after compression to 50%. The particles have a size uniformity of + 15% or more.

The particles include 1% or less polysaccharide. The polysaccharide is alginate. The alginate has a guluronic acid content of 60% or greater. The embolic particles are substantially insoluble in DMSO. The embolic particles are substantially free of animal-derived compounds. The polyvinyl alcohol is composed of substantially unmodified polyvinyl alcohol prepolymer. The polyvinyl alcohol is predominantly intrachain 1, 3-diols acetalized. The composition includes saline and/or contrast agent. The particles and/or composition are sterilized.

The gelling compound is a polysaccharide The gelling compound is alginate. The alginate has a guluronic acid content of 60% or more. The drops are contacted with a gelling agent. The gelling agent is a divalent cation. The cation is Ca+2. The base polymer is PVA. The PVA is reacted by acetalization. The PVA has a molecular weight of about 75,000 g/mole or greater. The viscosity of the base polymer and gelling compound is modified prior to forming said drops. The viscosity is modified by heating. The drops are formed by vibratory nebulization.

Administration is by percutaneous injection. Administration is by a catheter. The particles are introduced to the body through a lumen, and the lumen has a smaller diameter than the particles. The composition is used for treatment of uterine fibroids. The composition is used for treatment of tumors, including hypervascular tumors and for arteriovenous malformations (AVMs).

Some disorders or physiological conditions can be mediated by delivery of embolic compositions. Embolic compositions can be used, for example, in treatment of fibroids, internal bleeding AVMs and hypervascular tumors. Fibroids can include uterine fibroids which grow within the uterine wall, on the outside of the uterus, inside the uterine cavity, between the layers of broad ligament supporting the uterus, attached to another organ or on a mushroom-like stalk. Internal bleeding includes gastrointestinal, urinary, renal and varicose bleeding. AVMs are, for example, abnormal collections of blood vessels which shunt blood from a high pressure artery to a low pressure vein, resulting in hypoxia and malnutrition of those regions from which the blood is diverted.

Spherical embolic particles in the embolic compositions can be tailored to a particular application by varying particle size, porosity gradient, compressibility, sphericity and density of the particles. The uniform size of the spherical embolic particles can, for example, fit through the aperture of a catheter for administration by injection to a target site without partially or completely plugging the lumen of the catheter. The spherical embolic particles have a diameter of 1200 micron or less. Size uniformity of ± 15% of the spherical embolic particles allows the particles to stack evenly in the cylindrical lumen of the blood vessel to completely occlude the blood vessel lumen. Suspensions containing the embolic particles at density of 1.1 to 1.4 g/cm³ can be prepared in calibrated concentrations of the embolic particles for ease of delivery by the physician without rapid settlement of the suspension. Control in sphericity and uniformity of the embolic particles can result in reduction in aggregation caused, for example, by surface interaction of the particles. In addition, the embolic particles are relatively inert in nature.

The details of one or more embodiments of the invention are set forth in the accompanying drawings and the description below. Other features, objects, and advantages of the invention will be apparent from the description and drawings, and from the claims.

### DESCRIPTION OF DRAWINGS

**FIG. 1A** is a schematic illustrating injection of an embolic composition including embolic particles into a vessel, while **FIG. 1B** is a greatly enlarged view of the region A in **FIG. 1A****;**
**FIG.** 2A is a light micrograph of a collection of hydrated embolic particles, while **FIG. 2B** is a scanning electron microscope (SEM) photograph of the embolic particle surface and **FIGS. 2C-2E** are cross-sections of embolic particles;
**FIG. 3A** is a schematic of the manufacture of an embolic composition while **FIG. 3B** is an enlarged schematic of region A in **FIG. 3A**;
**FIG. 4** is a photograph of gel-stabilized drops;
**FIG. 5** is a graph of embolic particle size uniformity; and
**FIG. 6** is a schematic of an injection pressure testing equipment;
**FIG. 7** is an infrared spectrum of embolic particles.

Like reference symbols in the various drawings indicate like elements.

### DETAILED DESCRIPTION

### Composition

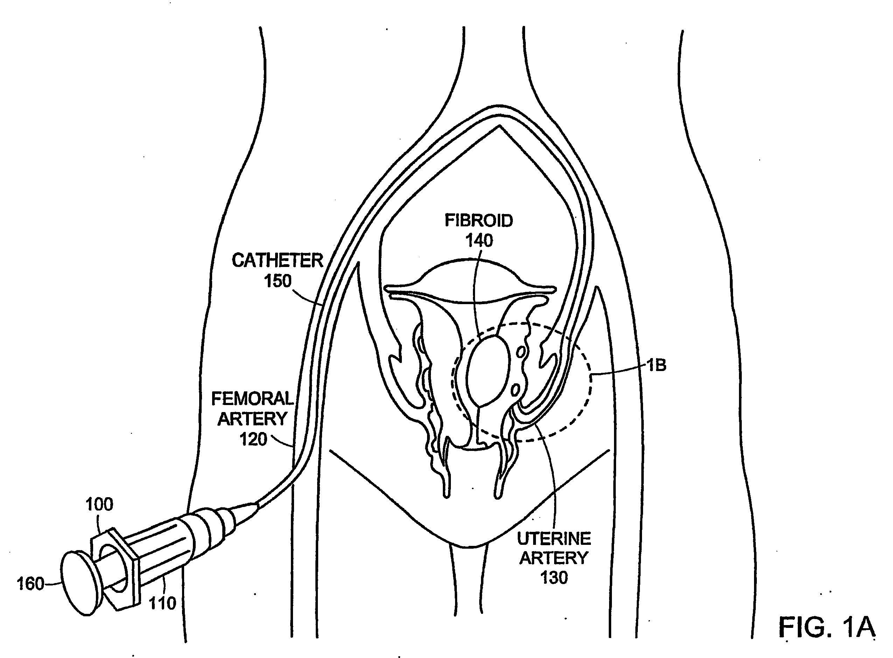

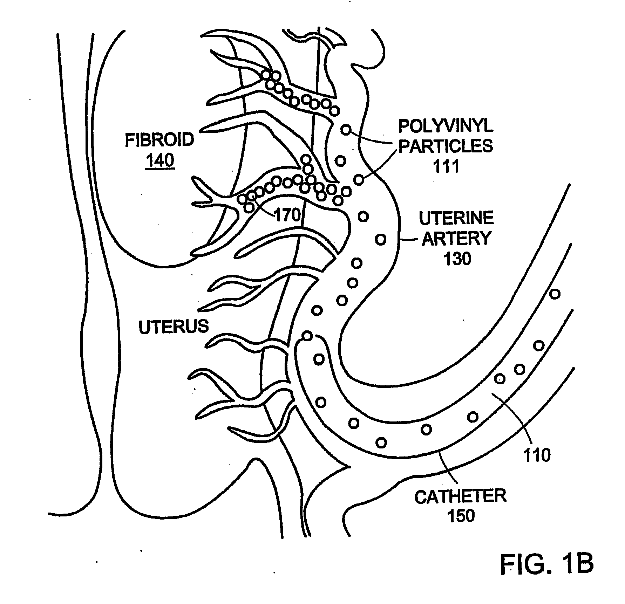

Referring to **FIGS. 1A** and **1B****,** an embolic composition **100,** including embolic particles **111** and carrier fluid, is injected into a vessel through an instrument such as a catheter **150.** The catheter is connected to a syringe barrel **110** with a plunger **160.** The catheter **150** is inserted, for example, at the leg of a patient into a femoral artery **120** to deliver the embolic composition **100** to, for example, occlude a uterine artery **130** leading to a fibroid **140.** The fibroid **140** is located in the uterus of a female patient. The embolic composition **100** is initially loaded into the syringe **110.** The plunger **160** of syringe **110** is compressed to deliver the embolic composition **100** through the catheter into lumen of the uterine artery **130.**

Referring particularly to **FIG. 1B** which is an enlarged view of section A of **FIG. 1A****,** the uterine artery **130** is subdivided into smaller uterine vessels **170** (about 2 mm or less) which feed a uterine fibroid **180.** The embolic particles **111** in embolic composition **100** partially or totally fill the lumen of uterine artery **130,** either partially or completely occluding the lumen of the uterine artery **130** feeding the uterine fibroid **140.**

The particles are substantially formed of polymer such as a highly water insoluble, high molecular weight polymer. As will be discussed below, a preferred polymer is high molecular weight polyvinyl alcohol (PVA) that has been acetalized. Preferably, the embolic particles are substantially pure intrachain 1,3 acetalized PVA and substantially free of animal derived residue such as collagen. In embodiments, the particles include a minor amount, e.g. less than 0.2 weight %, of alginate or another polysaccharide or gelling material.

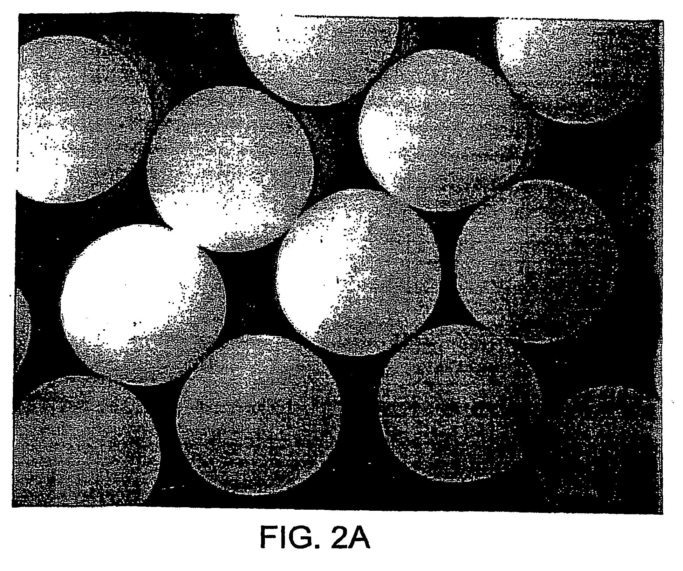

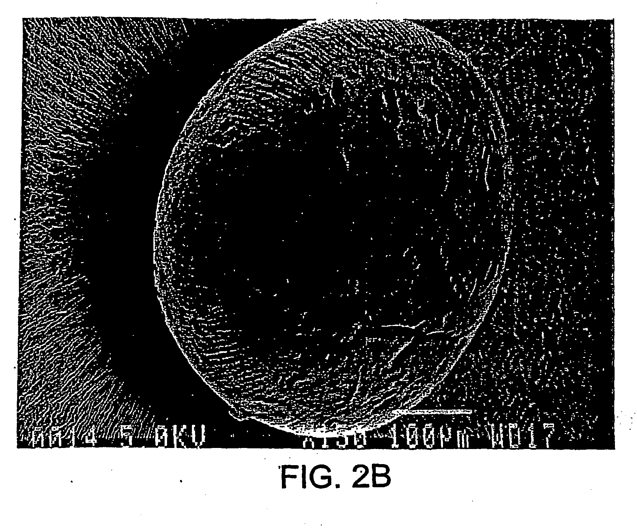

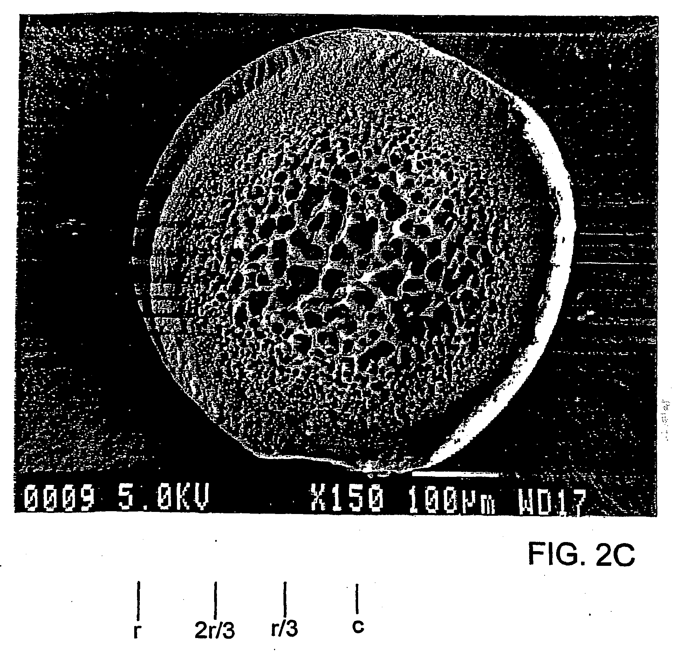

Referring to **FIG. 2A****,** embolic particles **111** have a substantially uniform spherical shape and size. Referring to **FIG. 2B****,** each embolic particle has a well-defined outer spherical surface including relatively small, randomly located pores. The surface appears substantially smooth, with some larger surface morphology such as crevice-like features. Referring to **FIGS. 2C-2E****,** SEM images of cross-sections through embolic particles, the body of the particle defines pores which provide compressibility and other properties to the embolic composition. Pores near the center of the particle are relatively large and pores near the surface of the particle are relatively small.

The region of small pores near the periphery of the embolic particle is relatively stiff and incompressible, which enhances resistance to shear forces and abrasion. In addition, the variable pore size profile produces a symmetric compressibility and, it is believed, a compressibility profile such that the particles are relatively easily compressed from a maximum, at rest diameter to a smaller, compressed first diameter but compression to even smaller diameter requires substantially greater force. A variable compressibility profile is believed to be due to the presence of a relative weak, collapsible inter-pore wall structure in the center region where the pores are large, and a stiffer inter-pore wall structure near the surface of the particle, where the pores are more numerous and relatively small. The variable pore size profile also is believed to enhance elastic recovery after compression. The pore structure also influences the density of the embolic particles and the rate of carrier fluid or body fluid uptake.

The embolic particles can be delivered through a catheter having a lumen area that is smaller, e.g. 50% smaller or less, than the uncompressed cross-sectional area of the particles. As a result, the embolic particles must be compressed to pass through the catheter for delivery into the body. The compression force is provided indirectly by increasing the pressure applied to the carrier fluid by depressing the syringe plunger. The embolic particles are relatively easily compressed to diameters sufficient for delivery through the catheter into the body. The robust, rigid surface region resists abrasion when the embolic particles contact hard surfaces such as syringe surfaces, hard plastic or metal stopcock surfaces, and the catheter lumen wall (e.g. Teflon) during delivery. Once in the body, the embolic particles substantially recover to original diameter and shape for efficient transport in the carrier and body fluid stream. At the point of occlusion, the particles can again compress as they aggregate in the occlusion region. The embolic particles form a dense occluding mass. The compression in the body is determined by the force provided by body fluid flow in the lumen. The compression may be limited by the compression profile of the particles and the number of embolic particles needed to occlude a given diameter may be reduced.

In embodiments, the particles have a diameter of 1500 or 1200 microns or less, and 10 microns or more, e.g. 400 microns or more and the pores are 50 or 35 to 0.01 micron. The embolic particles can be classified in size ranges of 500-700 microns, 700-900 microns, or 900-1200 microns. The particles typically have a mean diameter in approximately the middle of the range and variance of 20% or less, e.g. 15% or 10% or less.

Referring particularly to **FIG. 2C**, the particles can be considered to include a center region, C, from the center of the particle to a radius of r/3, a body region, B, from r/3 to 2 r/3 and a surface region, S, from 2r/3 to r. The regions can be characterized by the relative size of the pores and the number of pores of given sizes. In embodiments, the center region has a greater number of relatively large pores than the body region and the surface region. The large pores are in the range of 20 micron or more, e.g. 30 micron or more, or in the range of 20 to 35 micron. The body region has a greater number of intermediate size pores than the surface region. The intermediate size pores are in the range of 5 to 18 micron. In embodiments, the regions may also have different densities, with the density of the surface region being greater than the density of the body region, and the density of the body region being greater than the density of the center region.

The size of the pores in each of the regions can also be characterized by a distribution. In embodiments, the predominant pore size(s) in the center region being greater than the predominant pore size(s) in the body region and the predominant pore size(s) in the body region is greater than the predominant pore size(s) in the surface region. In embodiments, in the predominant pore size in the center region is 20 micron or more, e.g. 30 microns or more, or in the range of 20 to 35 microns. The predominant pore size in the body region is 18 micron or less, e.g. 15 micron or less, or in the range of 18 to 2 micron. The pores in the surface region are preferably predominantly less than 1 micron, e.g. 0.1 to 0.01 micron.

In embodiments, the predominant pore size in the body region is 50 to 70% of the pore size in the center region and the pore size in the surface region is 10% or less, e.g. 2% of the pore size in the body region. The size of the pores on the outer surface of the particle is predominantly in the range of 1 micron or less, e.g. 0.1 or 0.01 micron. In embodiments, the surface and/or surface region is substantially free of pores having a diameter larger than 10 micron or larger than 1 micron. In embodiments, the predominant pore size is in the region 0.8 or 0.9r to r is 1 micron or less, e.g. 0.5 to 0.1 micron or less. The region from the center of the particle to 0.8 or 0.9r has pores of 10 micron or greater and/or has a predominant pore size of 2 to 35 micron. In embodiments, the predominant pore size in the region 0.8 or 0.9r to r is 5% or less, e.g. 1% or 0.3% or less than the predominant pore size in the region from the center to 0.9r. the largest pores in the particles can have a size in the range of 1% or 5% or 10% or more of the particle diameter.

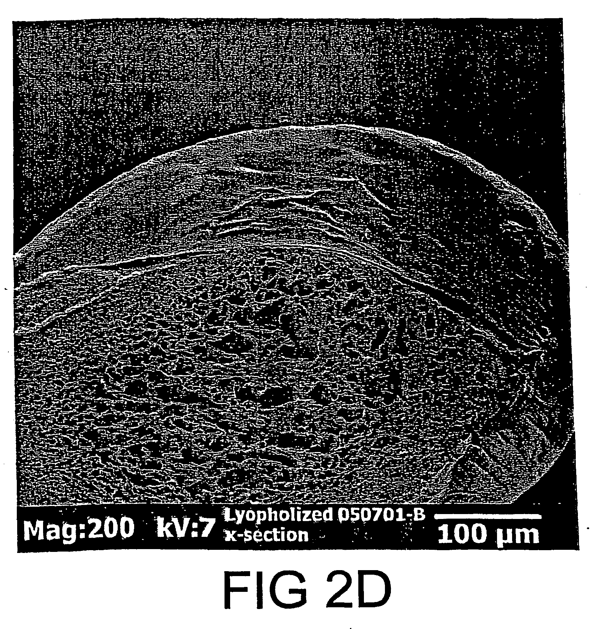

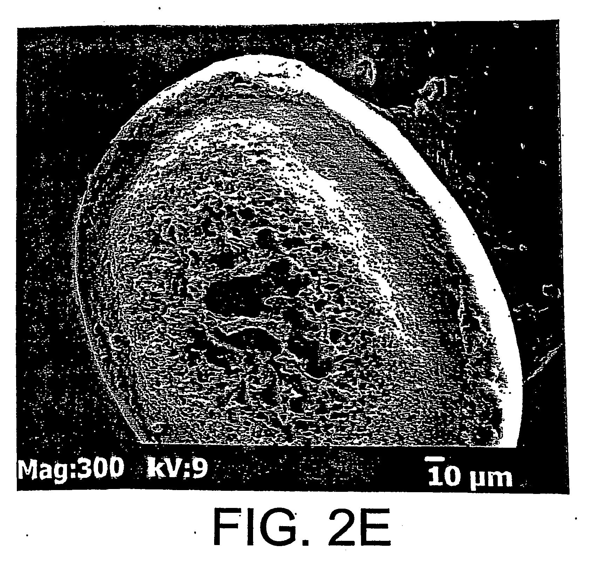

The size of the pores can be measured by viewing a cross-section as in Fig. 2C. For irregularly shaped pores, the maximum visible cross-section is used. The predominant pore size(s) can be found by measuring the size of the visible pores and plotting the number of pores as a function of size. The predominant pore size(s) are the sizes that are about the maximum in the distribution. In Fig. 2C, the SEM was taken on wet particles including absorbed saline, which were frozen in liquid nitrogen and sectioned. (Fig. 2B was taken prior to sectioning.) In Figs. 2D and 2E, the particle was freeze-dried prior to sectioning and SEM analysis.

The density of the particles is such that they are readily suspended in the carrier fluid such as a mixture of saline and contrast solution and remain suspended during delivery. In embodiments, the density is in 1.1 - 1.4g/cm³. For suspension in a saline-contrast solution, the density is 1.2 - 1.3g/cm³. The sphericity after compression in a catheter to 50% or more of their cross-sectional area is 0.90 or 0.95 or greater. In embodiments, the particles can be manually compressed, essentially flattened, while wet to less than 50% of original diameter and then, upon exposure to fluid, regain a sphericity of about 0.9 or more. The carrier fluid is a pharmaceutically acceptable carrier such as saline or contrast agent. The particles can be sterilized prior to use.

### Manufacture

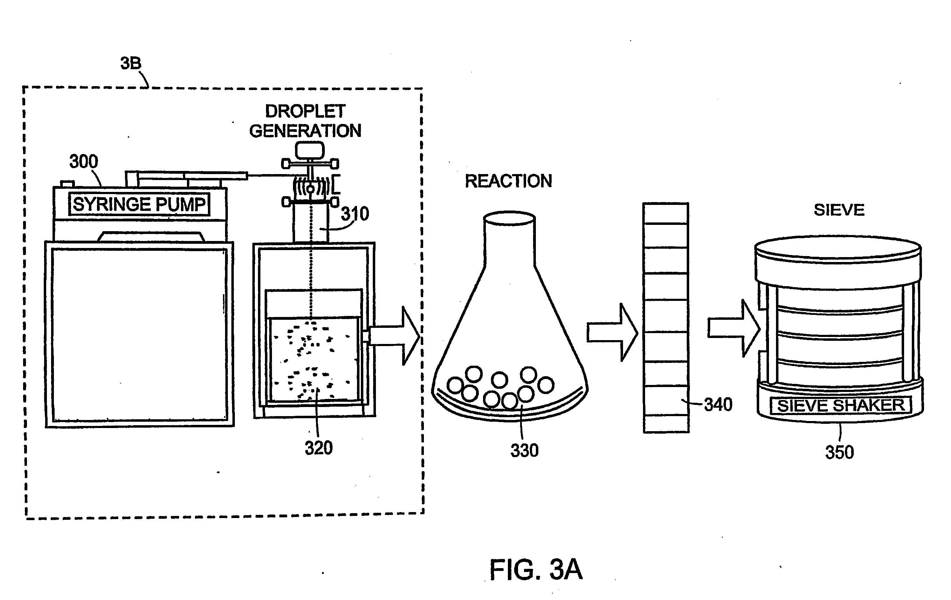

Referring to **FIG. 3A****,** a system for producing embolic particles includes a flow controller **300**, a drop generator **310,** a gelling vessel **320,** a reactor vessel **330**, a gel dissolution chamber **340** and a filter **350.** The flow controller **300** delivers polymer solutions to a viscosity controller **305,** which heats the solution to reduce viscosity prior to delivery to the drop generator **310.** The drop generator **310** forms and directs drops into a gelling vessel **320,** where drops are stabilized by gel formation. The gel-stabilized drops are transferred from the gelling vessel **320** to reactor vessel **330** where the polymer in the gel-stabilized drops are reacted forming precursor particles. The precursor particles are transferred to a gel dissolution chamber **340,** where the gel is dissolved. The particles are then filtered in a filter **350** to remove debris, sterilized, and packaged as an embolic composition including embolic particles.

A base polymer and a gelling precursor are dissolved in water and mixed. The mixture is introduced to a high pressure pumping apparatus, such as a syringe pump (e.g., model PHD4400, Harvard Apparatus, Holliston, MA). Examples of base polymers include polyvinyl alcohol, polyacrylic acid, polymethacrylic acid, poly vinyl sulfonate, carboxymethyl cellulose, hydroxyethyl cellulose, substituted cellulose, polyacrylamide, polyethylene glycol, polyamides, polyureas, polyurethanes, polyester, polyethers, polystyrene, polysaccharide, polylactic acid, polyethylene, polymethylmethacrylate and copolymers or mixtures thereof. A preferred polymer is polyvinyl alcohol. The polyvinyl alcohol, in particular, is hydrolyzed in the range of 80 to 99%. The weight average molecular weight of the base polymer can be in the range of 9000 to 186,000, 85,000 to 146,000 or 89,000 to 98,000. Gelling precursors include, for example, alginates, alginate salts, xanthan gums, natural gum, agar, agarose, chitosan, carrageenan, fucoidan, furcellaran, laminaran, hypnea, eucheuma, gum arabic, gum ghatti, gum karaya, gum tragacanth, hyalauronic acid, locust beam gum, arabinogalactan, pectin, amylopectin, other water soluble polysaccharides and other ionically crosslinkable polymers. A particular gelling precursor is sodium alginate. A preferred sodium alginate is high guluronic acid, stem-derived alginate (e.g. 50 or 60% or more guluronic acid with a low viscosity e.g. 20 to 80 mPa.s at 20°C) which produces a high tensile, robust gel. High molecular weight PVA is dissolved in water by heating, typically above 70°C, while alginates can be dissolved at room temperature. The PVA can be dissolved by mixing PVA and alginate together in a vessel which is heated to autoclave temperature (about 121°C). Alternatively, the PVA can be disposed in water and heated and the alginate subsequently added at room temperature to avoid exposing the alginate to high temperature. Heat can also be applied by microwave application. In embodiments, for PVA/alginate, the mixture is typically 7.5 to 8.5%, e.g. 8% by weight PVA and 1.5 to 2.5%, e.g. 2%, by weight alginate.

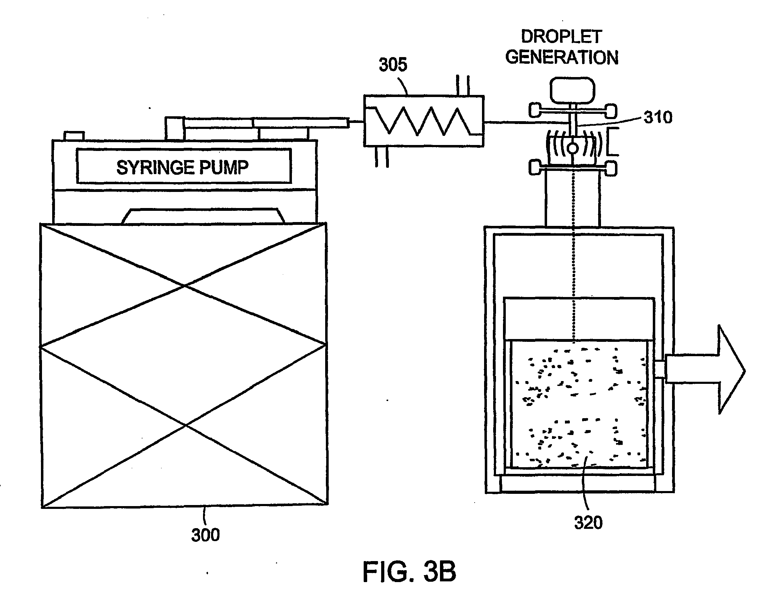

Referring to **FIG. 3B****,** the viscosity controller **305** is a heat exchanger circulating water at a predetermined temperature about the flow tubing between the pump and drop generator. The mixture of base polymer and gelling precursor flows into the viscosity controller **305,** where the mixture is heated so that its viscosity is lowered to a level for efficient formation of very small drops. For a high molecular weight PVA/alginate solution, the temperature of the circulating water is less than about 75°C and more than about 60°C, for example, 65°C which maintains the mixture at a viscosity of 90-200 mPa.s. For spherical particles, the viscosity of the drops is maintained so they are captured in the gelling vessel without splintering or cojoining which can create irregular, fiberous particles. In other embodiments, the flow controller and/or the drop generator can be placed in a temperature-controlled chamber, e.g. an oven, or a heat tape wrap, to maintain a desired viscosity.

The drop generator **310** generates substantially spherical drops of predetermined diameter by forcing a stream of the mixture of base polymer and gelling precursor through a nozzle which is subject to a periodic disturbance to break up the jet stream into drops. The jet stream can be broken into drops by vibratory action generated for example, by an electrostatic or piezoelectric element. The drop size is controlled by controlling the flow rate, viscosity, amplitude, and frequency at which the element is driven. Lower flow rates and higher frequencies produce smaller drops. A suitable electrostatic drop generator is available from NISCO Engineering, model NISCO Encapsulation unit VAR D, Zurich, Switzerland. In embodiments, the frequency is in the range of 0.1 to 0.8 kHz. The flow rate through the droplet generator is in the range of about 1 to 12 mL per minute. The drop generator can include charging the drops after formation such that mutual repulsion between drops prevents drop aggregation as drops travel from the generator to the gelling vessels. Charging may be achieved by, e.g. an electrostatic charging device such as a charged ring positioned downstream of the nozzle.

Drops of the base polymer and gelling precursor mixture are captured in the gelling vessel **320**. The gelling vessel **320** contains a gelling agent which interacts with the gelling precursor to stabilize drops by forming a stable gel. Suitable gelling agents include, for example, a divalent cation such as alkali metal salt, alkaline earth metal salt or a transition metal salt that can ionically crosslink with the gelling agent. An inorganic salt, for example, a calcium, barium, zinc or magnesium salt can be used as a gelling agent. In embodiments, particularly those using an alginate gelling precursor, a suitable gelling agent is calcium chloride. The calcium cations have an affinity for carboxylic groups in the gelling precursor. The cations complex with carboxylic groups in the gelling precursor resulting in encapsulation of the base polymer in a matrix of gelling precursor.

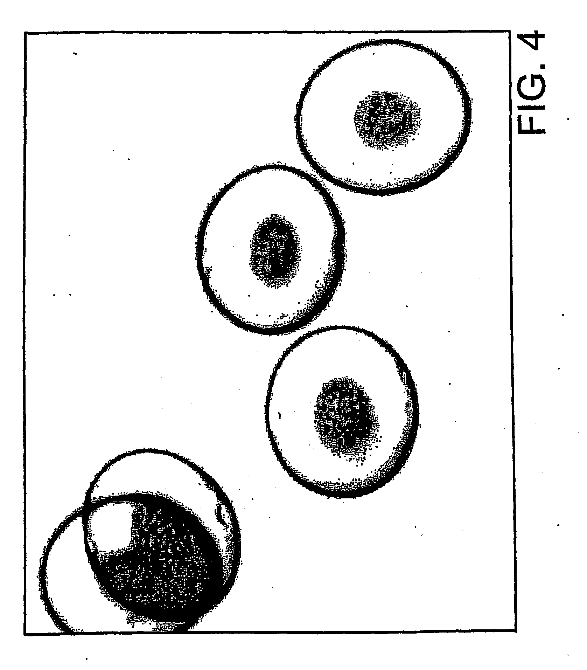

Referring to **FIG. 4**, a photo-image of the gelled particles, the gelling agent is in an amount selected in accordance with the desired properties of the particles. As evident, a pore structure in the particle forms in the gelling stage. The concentration of the gelling agent can control pore formation in the particle, thereby controlling the porosity gradient in the embolic particle. Adding non-gelling ions, for example, sodium ions, to the gelling solution can reduce the porosity gradient, resulting in a more uniform intermediate porosity throughout the particle. In embodiments, the gelling agent is, for example, 0.01-10 weight percent, 1-5 weight percent or 2 weight percent in deionized water. In embodiments, particles, including gelling agent and a pore structure can be used in embolic compositions.

Following drop stabilization, the gelling solution is decanted from the solid drops and the stabilized drops are transferred to the reactor vessel **330**. In the reactor vessel **330**, the stabilized drops are reacted to produce precursor particles. The reactor vessel includes an agent that chemically reacts with the base polymer, e.g. to cause crosslinking between polymer chains and/or within a polymer chain. The agent diffuses into the stabilized drops from the surface of the particle in a gradient which, it is believed, provides more crosslinking near the surface of the stabilized drop compared to the body and center of the drop. Reaction is greatest at the surface of the drop, providing a stiff, abrasion resistant exterior. For polyvinyl alcohol, for example, the vessel **330** includes aldehydes, such as formaldehyde, glyoxal, benzaldehyde, aterephthalaldehyde, succinaldehyde and glutaraldehyde for the acetalization of polyvinyl alcohol. The vessel **330** also includes an acid, for example, strong acids such as sulfuric acid, hydrochloric acid, nitric acid and weak acids such as acetic acid, formic acid and phosphoric acid. In embodiments, the reaction is primarily a 1,3 acetalization:

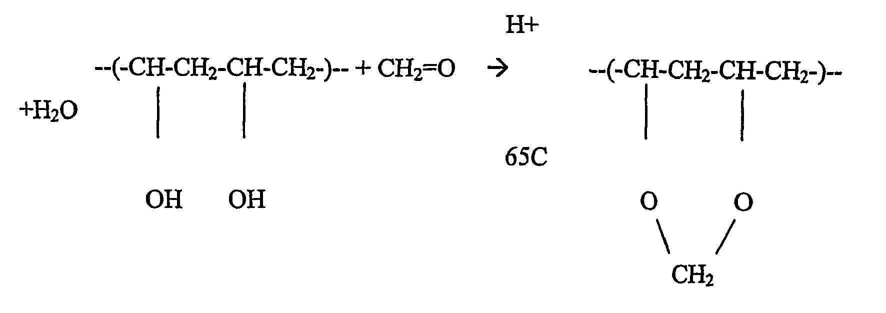

This intra-chain acetalization reaction can be carried out with relatively low probability of inter-chain crosslinking as described in John G. Pritchard "Poly(Vinyl Alcohol) Basic Properties And Uses (Polymer Monograph, vol. 4) (see p. 93-97), Gordon and Breach, Science Publishers LTD., London, 1970, the entire contents of which is hereby incorporated by reference. Some OH groups along a polymer chain may remain unconverted since the reaction proceeds in a random fashion and there will be left over OH groups that do not react with adjacent groups.

Adjusting the amount of aldehyde and acid used, reaction time and reaction temperature can control the degree of acetalization. In embodiments, the reaction time is e.g., 5 minutes to 1 hour, 10 to 40 minutes or 20 minutes. The reaction temperature can be 25°C to 150°C or 75°C to 130°C or 65°C. The reactor vessel is placed in a water bath fitted with a orbital motion mixer. The crosslinked precursor particles are washed several times with deionized water to neutralize the particles and remove any residual acidic solution.

The precursor particles are transferred to the dissolution chamber **340** to remove the gelling precursor, e.g. by an ion exchange reaction. In embodiments, sodium alginate is removed by ion exchange with a solution of sodium hexa-metaphosphate (EM Science). The solution can include, for example, ethylenediaminetetracetic acid (EDTA), citric acid, other acids and phosphates. The concentration of the sodium hexa-metaphosphate can be, for example, 1-20 weight %, 1-10 weight % or 5 weight % in deionized water. Residual gelling precursor, for example, sodium alginate, can be determined by assay for detection of uronic acids in, for example, alginates containing mannuronic and guluronic acid residues. Suitable assays include rinsing the particles with sodium tetraborate in sulfuric acid solution to extract alginate and combining the extract with metahydroxydiphenyl colormetric reagent and determining concentration by UV/VIS spectroscopy. Testing can be carried out by alginate suppliers such as FMC Biopolymer, Oslo, Norway. Residual alginate may be present in the range of 20-35% by weight prior to rinsing and in the range of 0.01-0.5% or 0.1-0.3% or 0.18% in the particles after rinsing for 30 minutes in water at about 23°C.

The particles are filtered through filter **350** to remove residual debris. Particles of 500 to 700 microns are filtered through a sieve of 710 microns and then a sieve of 300 microns. Particles of 700 to 900 microns are filtered through a sieve of 1000 microns and then a sieve of 500 microns. Particles of 900 to 1200 microns are filtered through a sieve of 1180 microns and then a sieve of 710 microns.

The filtered particles are sterilized by a low temperature technique such as e-beam irradiation, and packaged, typically 1 to 5 ml of particles in 5 to 10 ml saline. In embodiments, electron beam irradiation can be used to pharmaceutically sterilize the particles to reduce bioburden. In e-beam sterilization, an electron beam is accelerated using magnetic and electric fields, and focused into a beam of energy. This resultant beam can be scanned by means of an electromagnet to produce a "curtain" of accelerated electrons. The accelerated electron beam penetrates the collection of embolic particles to confer upon them electrons which destroy bacteria and mold to sterilize and reduce the bioburden in the embolic particles. Electron beam sterilization can be carried out by sterilization vendors such as Titan Scan, Lima, Ohio.

### Examples

### Example 1

Embolic particles are manufactured from an aqueous solution containing 8 weight % of polyvinyl alcohol, 99+% hydrolyzed, average M_{w} 89,000-120,000 (ALDRICH) and 2 weight% of gelling precursor, sodium alginate, PRONOVA UPLVG, (FMC BioPolymer, Princeton, NJ) in deionized water and the mixture is heated to about 121° C. The solution has a viscosity of about 310 mPa.s at room temperature and a viscosity of about 160 mPa.s at 65°C. Using a syringe pump (Harvard Apparatus), the mixture is fed to drop generator (Nisco Engineering). Drops are directed into a gelling vessel containing 2 weight % of calcium chloride in deionized water and stirred with a stirring bar. The calcium chloride solution is decanted within about three minutes to avoid substantial leaching of the polyvinyl alcohol from the drops into the solution. The drops are added to the reaction vessel containing a solution of 4% by weight of formaldehyde (37 wt% in methanol) and 20% by weight sulfuric acid (95-98% concentrated). The reaction solution is stirred at 65°C for 20 minutes. Precursor particles are rinsed with deionized water (3 X 300 mL) to remove residual acidic solution. The sodium alginate is substantially removed by soaking the precursor particles in a solution of 5 weight % of sodium hexa-methaphosphate in deionized water for 0.5 hour. The solution is rinsed in deionized water to remove residual phosphate and alginate. The particles are filtered by sieving, as discussed above, placed in saline (USP 0.9% NaCI) and followed by irradiation sterilization.

Particles were produced at the nozzle diameters, nozzle frequencies and flow rates (amplitude about 80% of maximum) described in Table I.

**TABLE 1**

| Bead Size (microns) | Nozzle Diameter (microns) | Frequency (kHz) | Flow Rate (mL/min) | Density (g/mL) | Sphericity | Suspendability (minutes) |
|---|---|---|---|---|---|---|
| 500-700 | 150 | 0.45 | 4 | - | 0.92 | 3 |
| 700-900 | 200 | 0.21 | 5 | 1.265 | 0.94 | 5 |
| 900-1200 | 300 | 0.22 | 10 | - | 0.95 | 6 |

Suspendability is measured at room temperature by mixing a solution of 2 ml of particles in 5 ml saline with contrast solution (Omnipaque 300, Nycomed, Buckinghamshire, UK) and observing the time for about 50% of the particles to enter suspension, i.e. have not sunk to the bottom or floated to the top of a container (about 10 ml, 25 mm dia vial). Suspendability provides a practical measure of how long the particles will remain suspended in use. (Omnipaque is an aqueous solution of Iohexol, N.N.-Bis (2,3-dihydroxypropyl)-T-[N-(2,3-dihydroxypropyl)-acetamide]-2,4,6-trilodoisophthalamide; Omnipaque 300 contains 647 mg of iohexol equivalent to 300 mg of organic iodine per ml. The specific gravity of 1.349 of 37° C and an absolute viscosity 11.8 mPa.s at 20° C.) The particles remain in suspension for 2 to 3 minutes.

Particle size uniformity and sphericity is measured using a Beckman Coulter RapidVUE Image Analyzer version 2.06 (Beckman Coulter, Miami, FL). Briefly, the RapidVUE takes an image of continuous-tone (gray-scale) form and converts it to a digital form through the process of sampling and quantization. The system software identifies and measures particles in an image in the form of a fiber, rod or sphere. Sphericity computation and other statistical definitions are in Appendix A, attached, which is a page from the RapidVUE operating manual.

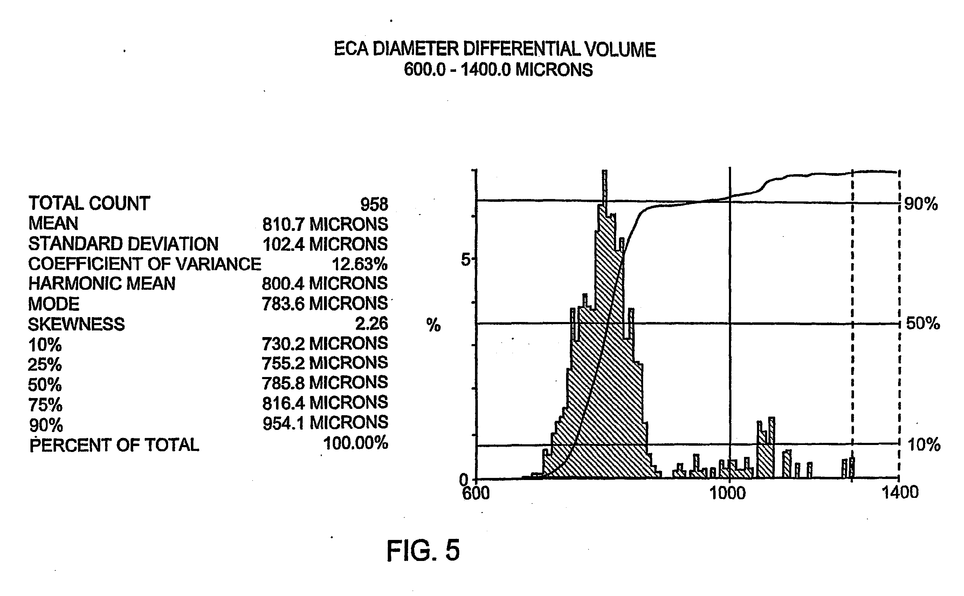

Referring to **FIG. 5**, particle size uniformity is illustrated for particles 700 - 900 micron. The x-axis is the particle diameter. The y-axis is the volume normalized percentage of particles at each particle size. The total volume of particles detected is computed and the volume of the particles at each diameter is divided by the total volume. The embolic particles have distribution of particle sizes with variance of less than ± 15%.

### Example 2

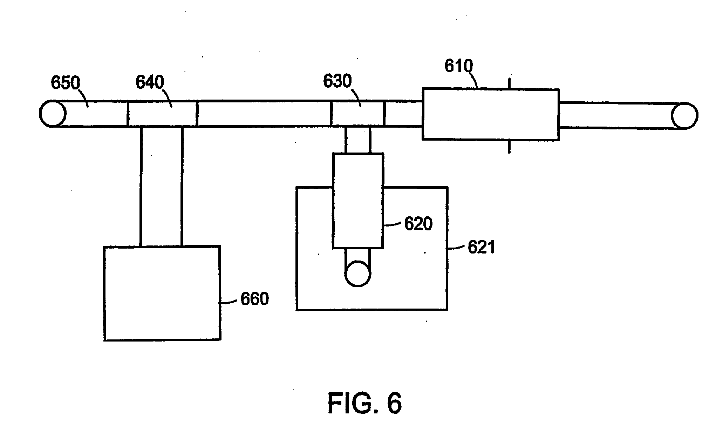

Referring to **FIG. 6**, a catheter compression test investigates the injectability, and indirectly, the compressibility of the particles. The test apparatus includes a reservoir syringe **610** and an injection syringe **620** coupled to a T-valve **630.** Syringe **610** is a 20mL syringe while injection syringe **620** is a 3 mL syringe. T-valve **630** is coupled in series to a second T-valve **640.** T-valve **640** is coupled to a catheter **650** and a pressure transducer **660.** Injection syringe **620** is coupled to a syringe pump **621** (Harvard Apparatus).

To test deliverability of the particles, syringe **610** and syringe **620** are loaded with embolic composition in saline and contrast (50/50 Ominipaque 300). The embolic composition in syringes **610** and **620** is intermixed by turning the T-valve to allow fluid between the syringes to mix and suspend the particles. After mixing, the embolic composition in syringe **620** flows at a rate of about 10mL/min. The back pressure generated in the catheter **650** is measured by the pressure transducer **670** in millivolts to measure the clogging of catheter **650.** About 1 ml of the particles is mixed in 10mL of solution.

Results for several different catheters (available from Boston Scientific, Natick, MA) and particle sizes are shown in Table 2. The baseline pressure is the pressure observed when injecting carrier fluid only. The delivery pressure is the pressure observed while delivering particles in carrier fluid. The average is the average of the peak pressure observed in the three runs.

| SIZE (microns) | Delivery Catheter | Inner Diameter (inches) | Avg. Baseline Pressure (psia) | Avg. Delivery Pressure (psia) | Total number of Clogs |
|---|---|---|---|---|---|
| 500-700 | RENEGADE ® | 0.021 (533 micron) | 32.610 | 33.245 | 0 |
| 700-900 | FASTRACKER® | 0.024 (609 micron) | 11.869 | 13.735 | 0 |
| 900-1200 | GLIDECATH ® | 0.038 (965 micron) | 0.788 | 0.864 | 0 |

As evident, particles in each of the size ranges were successfully delivered without clogging through catheters having a lumen diameter smaller than the largest particle size. The particles exhibit a post-compression sphericity of about 0.9 or more.

### Example 4

Solubility is tested by mixing particles in a solution of solvent at room temperature for about 0.5 hour and observing the mixture for visible signs of dissolution. The particles are insoluble in DMSO (Dimethylsulfoxide), HFIP (Hexafluom-isopropanol), and THF (Tetrahydrafuran).

### Example 5

Embolic particles include the following glass transition temperatures as measured by differential scanning calorimetry data (DSC)

| Product | 500-700 microns | 900-1200 microns |
|---|---|---|
| Glass transition temperature (Tg) | 109.30-110.14 | 108.30-111.87 |

### Example 6

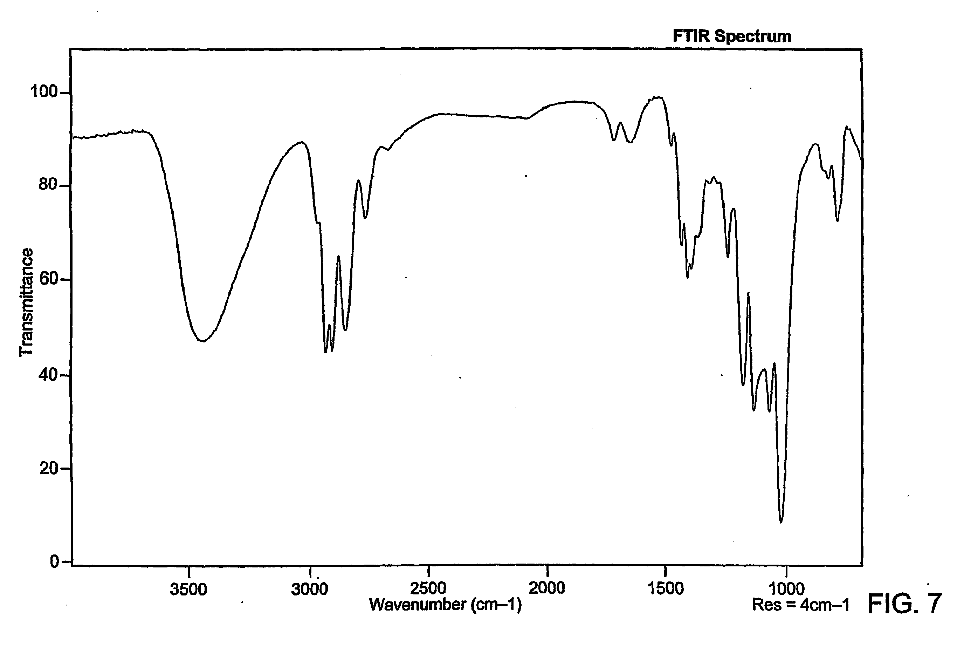

Referring to Fig. 7, an ATR infrared spectrum of dried particles is provided.

### Use

The embolic compositions can be used as pharmaceutically acceptable compositions in the treatment of, for example, fibroids, tumors, internal bleeding, AVMs, hypervascular tumors, fillers for aneurysm sacs, endoleak sealants, arterial sealants, puncture sealants and occlusion of other lumens such as fallopian tubes. Fibroids can include uterine fibroids which grow within the uterine wall (intramural type), on the outside of the uterus (subserosal type), inside the uterine cavity (submucosal type), between the layers of broad ligament supporting the uterus (interligamentous type), attached to another organ (parasitic type), or on a mushroom-like stalk (pedunculated type). Internal bleeding includes gastrointestinal, urinary, renal and varicose bleeding. AVMs are for example, abnormal collections of blood vessels, e.g. in the brain, which shunt blood from a high pressure artery to a low pressure vein, resulting in hypoxia and malnutrition of those regions from which the blood is diverted.

The magnitude of a therapeutic dose of the embolic composition can vary based on the nature, location and severity of the condition to be treated and the route of administration. A physician treating the condition, disease or disorder can determine effective amount of embolic composition. An effective amount of embolic composition refers to the amount sufficient to result in amelioration of symptoms or a prolongation of survival of the patient. The embolic compositions can be administered as pharmaceutically acceptable compositions to a patient in any therapeutically acceptable dosage, including those administered to a patient intravenously, subcutaneously, percutaneously, intratrachealy, intramuscularly, intramucosaly, intracutaneously, intraarticularly, orally or parenterally.

Compositions containing the embolic particles can be prepared in calibrated concentrations of the embolic particles for ease of delivery by the physician. The density of the composition can be from 1.1 to 1.4 g/cm³, or from 1.2 to 1.3 g/cm³ in saline solution. Suspensions of the embolic particles in saline solution can be prepared to form stable suspensions over duration of time. The suspensions of embolic particles can be stable from 1 to 10 minutes, 2-7 minutes or 3 to 6 minutes. The physician can determine concentration of embolic particles by adjusting the weight ratio of the embolic particles to physiological solution. If weight ratio of the embolic particles is too small, too much liquid could be injected in a blood vessel, possibly allowing the embolic particles to stray into lateral vessels. In embodiments, the weight ratio of the embolic particles to the physiological solution is 0.01 to 15% by weight. The embolic composition can include a mixture of particles including particles with the pore profiles discussed above and particles with other pore profiles or non-porous particles. Particles can be used for embolic applications without removal of the gelling agent (e.g. alginate) for example at the stabilized drop stage or precursor particle stages described above. While substantially spherical particles are preferred, non-spherical particles can be manufactured and formed by controlling, e.g. drop formation conditions or by post-processing the particles, e.g. by cutting or dicing into other shapes. Particles can also be shaped by physical deformation followed by crosslinking. Particle shaping is described in U.S. Serial No. 10/116,330 filed April 4, 2002.

Other embodiments are within the scope of the following claims.

## Claims

1. An embolic composition, comprising
spherical embolic particles having a diameter of 1200 micron or less and a sphericity of 90% or more, the particles comprising polyvinyl alcohol, **characterized in that** each particle includes an interior region having pores with a first predominant pore size of 10 microns or more, and a surface region surrounding the interior region and having pores with a second predominant pore size of 2 microns or less.

2. The composition of claim 1, wherein the first predominant pore size is 20 micron or more.

3. The composition of claim 1, wherein the first predominant pore size is 30 micron or more.

4. The composition of claim 1, wherein the surface region extends from 0.8 of the radius of the particle to the radius of the particle.

5. The composition of claim 1, wherein the surface region extends from 2/3 of the radius of the particle to the radius of the particle.

6. The composition of claim 1, wherein the predominant pore size generally, progressively increases from the surface region to the interior region of the particle.

7. The composition of claim 1, wherein the predominant pore size on a particle surface is 1 micron or less.

8. The composition of claim 5, wherein the second predominant pore size is in the range of 1 micron or less.

9. The composition of claim 8, wherein the second predominant pore size is 0.1 micron or less.

10. The composition of claim 13, wherein each particle has an interior region from the center of the particle to 1/3 of the radius of the particle in which the predominant pore size is 20 to 35 micron.

11. The composition of claim 1, wherein the surface region of each particle extends from 2/3 of the radius of the particle to the surface of the particle and the second predominant pore size is 10% or less than the first predominant pore size.

12. The composition of claim 1, wherein the particles have a density of 1.4 g/cm³.

13. The composition of claim 1, wherein the particles have a density of 1.2 to 1.3 g/cm³.

14. The composition of claim 1, wherein the particles have an initial sphericity of 97% or more.

15. The composition of claim 20, wherein the particles have a sphericity of 90% after compression to 50%.

16. The composition of claim 1, wherein the particles have a size uniformity of ± 15% or more.

17. The composition of claim 1, wherein the particles include 1% or less polysaccharide.

18. The composition of claim 17, wherein the polysaccharide is alginate.

19. The composition of claim 18, wherein the alginate has a guluronic acid content of 60% or greater.

20. The composition of claim 1, wherein the embolic particles are insoluble in DMSO.

21. The composition of claim 1, wherein the embolic particles are free of animal-derived compounds.

22. The composition of claim 1, wherein the polyvinyl alcohol is composed of unmodified polyvinyl alcohol prepolymer.

23. The composition of claim 1, wherein the polyvinyl alcohol is intrachain 1,3-diols acetalized.

24. The composition of claim 1, wherein the particles are in a pharmaceutically acceptable medium.

25. The composition of claim 30, wherein the medium comprises saline.

26. A method of manufacturing embolic particles, comprising:
generating drops comprising a base polymer and a gelling compound;
reacting the base polymer;
removing the gelling compound; and
combining the particles with a pharmaceutically acceptable medium.

27. The method of claim 26, wherein the gelling compound is a polysaccharide.

28. The method of claim 26, wherein the gelling compound is alginate.

29. The method of claim 28, wherein the alginate has a guluronic acid content of 60% or more.

30. The method of any one of claims 26 to 29 comprising contacting the drops with a gelling agent.

31. The method of claim 30, wherein the gelling agent is a divalent cation.

32. The method of claim 31, wherein the cation is Ca⁺².

33. The method of claim 26, wherein the base polymer is PVA.

34. The method of claim 33 comprising reacting the PVA by acetalization.

35. The method of claim 33 or 34, wherein the PVA has a molecular weight of 75,000 g/mole or greater.

36. The method of claim 26 comprising lowering the viscosity of the base polymer and gelling compound in forming said drops.

37. The method of claim 36 comprising lowering the viscosity by heating.

38. The method of claim 26 comprising forming said drops by vibratory nebulization.

## Patentansprüche

1. Embolische Zusammensetzung, umfassend:
sphärische embolische Partikel mit einem Durchmesser von 1200 Mikrometern oder weniger und einer Rundung von 90 % oder mehr, wobei die Partikel Polyvinylalkohol umfassen, **dadurch gekennzeichnet, dass** jeder Partikel einen Innenbereich mit Poren mit einer ersten vorherrschenden Porengröße von 10 Mikrometern oder mehr einschließt, und einen Oberflächenbereich, der den Innenbereich umgibt, und Poren mit einer zweiten vorherrschenden Porengröße von 2 Mikrometern oder weniger aufweist.

2. Zusammensetzung nach Anspruch 1, wobei die erste vorherrschende Porengröße 20 Mikrometer oder mehr beträgt.

3. Zusammensetzung nach Anspruch 1, wobei die erste vorherrschende Porengröße 30 Mikrometer oder mehr beträgt.

4. Zusammensetzung nach Anspruch 1, wobei sich der Oberflächenbereich von 0,8 des Radius des Partikels zu dem Radius des Partikels erstreckt.

5. Zusammensetzung nach Anspruch 1, wobei sich der Oberflächenbereich von 2/3 des Radius des Partikels zu dem Radius des Partikels erstreckt.

6. Zusammensetzung nach Anspruch 1, wobei sich die vorherrschende Porengröße im Allgemeinen zunehmend von dem Oberflächenbereich zu dem Innenbereich des Partikels vergrößert.

7. Zusammensetzung nach Anspruch 1, wobei die vorherrschende Porengröße auf einer Partikeloberfläche 1 Mikrometer oder weniger ist.

8. Zusammensetzung nach Anspruch 5, wobei die zweite vorherrschende Porengröße im Bereich von 1 Mikrometer oder weniger liegt.

9. Zusammensetzung nach Anspruch 8, wobei die zweite vorherrschende Porengröße 0,1 Mikrometer oder weniger ist.

10. Zusammensetzung nach Anspruch 13, wobei jeder Partikel einen Innenbereich vom Mittelpunkt des Partikels bis zu 1/3 des Radius des Partikels aufweist, in welchem die vorherrschende Porengröße 20 bis 35 Mikrometer beträgt.

11. Zusammensetzung nach Anspruch 1, wobei sich der Oberflächenbereich jedes Partikels von 2/3 des Radius des Partikels zu der Oberfläche des Partikels erstreckt, und die zweite vorherrschende Porengröße 10 % oder weniger als die erste vorherrschende Porengröße beträgt.

12. Zusammensetzung nach Anspruch 1, wobei die Partikel eine Dichte von 1,4 Gramm pro Kubikzentimeter aufweisen.

13. Zusammensetzung nach Anspruch 1, wobei die Partikel eine Dichte von 1,2 bis 1,3 Gramm pro Kubikzentimeter aufweisen.

14. Zusammensetzung nach Anspruch 1, wobei die Partikel eine ursprüngliche Rundheit von 97 Prozent oder mehr aufweisen.

15. Zusammensetzung nach Anspruch 14, wobei die Partikel eine Rundheit von 90 % nach einer Kompression auf 50 % aufweisen.

16. Zusammensetzung nach Anspruch 1, wobei die Partikel eine Größengleichmäßigkeit von ± 15 % oder mehr aufweisen.

17. Zusammensetzung nach Anspruch 1, wobei die Partikel 1 % oder weniger Polysaccharide einschließen.

18. Zusammensetzung nach Anspruch 17, wobei das Polysaccharid ein Alginat ist.

19. Zusammensetzung nach Anspruch 18, wobei das Alginat einen Guluron-Säure-Gehalt von 60 % oder mehr aufweist.

20. Zusammensetzung nach Anspruch 1, wobei die embolischen Partikel in DMSO unlöslich sind.

21. Zusammensetzung nach Anspruch 1, wobei die embolischen Partikel frei von tierischen Verbunden sind.

22. Zusammensetzung nach Anspruch 1, wobei der Polyvinylalkohol aus einem unmodifizierten Polyvinylalkohol-Vorpolymer besteht.

23. Zusammensetzung nach Anspruch 1, wobei der Polyvinylalkohol im Ketteninneren 1,3-Diol azetalisiert ist.

24. Zusammensetzung nach Anspruch 1, wobei sich die Partikel in einem pharmazeutisch akzeptablen Medium befinden.

25. Zusammensetzung nach Anspruch 24, wobei das Medium Saline umfasst.

26. Verfahren zur Herstellung embolischer Partikel, umfassend:
Erzeugen von Tropfen, die ein Basispolymer und einen Gelierverbund umfassen;
Einwirken des Basispolymers;
Entfernen des Gelierverbunds; und
Kombinieren der Partikel mit einem pharmazeutisch akzeptablen Medium.

27. Verfahren nach Anspruch 26, wobei der Gelierverbund Polysaccharid ist.

28. Verfahren nach Anspruch 26, wobei der Gelierverbund Alginat ist.

29. Verfahren nach Anspruch 28, wobei das Alginat einen Guluron-SäureGehalt von 60 % oder mehr aufweist.

30. Verfahren nach irgendeinem der Ansprüche 26 bis 29, umfassend das Kontaktieren der Tropfen mit einem Gelierzusatz.

31. Verfahren nach Anspruch 30, wobei der Gelierzusatz ein divalentes Kation ist.

32. Verfahren nach Anspruch 31, wobei das Kation Ca⁺² ist.

33. Verfahren nach Anspruch 26, wobei der Basispolymer PVA ist.

34. Verfahren nach Anspruch 33, umfassend das Einwirken des PVA durch Azetalisierung.

35. Verfahren nach Anspruch 33 oder 34, wobei das PVA ein Molekulargewicht von 75.000 g/Mol oder mehr aufweist.

36. Verfahren nach Anspruch 26, umfassend das Absenken der Viskosität des Basispolymers und des Gelierverbunds in Form von Tropfen.

37. Verfahren nach Anspruch 36, umfassend das Absenken der Viskosität durch Erwärmen.

38. Verfahren nach Anspruch 26, umfassend das Bilden von Tropfen durch Vibrationszerstäubung.

## Revendications

1. Une composition embolique, comprenant :
des particules emboliques sphériques ayant un diamètre de 1200 microns ou moins et une sphéricité de 90 % ou plus, les particules comprenant un alcool polyvinylique, **caractérisée en ce que** chaque particule comprend une région intérieure ayant des pores avec une première dimension de pore prédominante de 10 microns ou plus, et une région de surface entourant la région intérieure et ayant des pores avec une seconde dimension de pore prédominante de 2 microns ou moins.

2. La composition de la revendication 1, dans laquelle la première dimension de pore prédominante est de 20 microns ou plus.

3. La composition de la revendication 1, dans laquelle la première dimension de pore prédominante est de 30 microns ou plus.

4. La composition de la revendication 1, dans laquelle la région de surface s'étend depuis 0,8 fois le rayon de la particule jusqu'au rayon de la particule.

5. La composition de la revendication 1, dans laquelle la région de surface s'étend depuis les deux tiers du rayon de la particule jusqu'au rayon de la particule.

6. La composition de la revendication 1, dans laquelle de façon générale la dimension de pore prédominante augmente progressivement depuis la région de surface jusqu'à la région intérieure de la particule.

7. La composition de la revendication 1, dans laquelle la dimension de pore prédominante sur une surface de particule est de 1 micron ou moins.

8. La composition de la revendication 5, dans laquelle la seconde dimension de pore prédominante est dans la plage de 1 micron ou moins.

9. La composition de la revendication 8, dans laquelle la seconde dimension de pore prédominante est de 0,1 micron ou moins.

10. La composition de la revendication 9, dans laquelle chaque particule possède une région intérieure allant du centre de la particule jusqu'au tiers du rayon de la particule où la dimension de pore prédominante est de 20 à 35 microns.

11. La composition de la revendication 1, dans laquelle la région de surface de chaque particule s'étend depuis les deux tiers du rayon de la particule jusqu'à la surface de la particule et la seconde dimension de pore prédominante est de 10 % ou moins inférieure à la première dimension de pore prédominante.

12. La composition de la revendication 1, dans laquelle les particules ont une densité de 1,4 g/cm³.

13. La composition de la revendication 1, dans laquelle les particules ont une densité de 1,2 à 1,3 g/cm³.

14. La composition de la revendication 1, dans laquelle les particules ont une sphéricité initiale de 97 % ou plus.

15. La composition de la revendication 14, dans laquelle les particules ont une sphéricité de 90 % après compression à 50 %.

16. La composition de la revendication 1, dans laquelle les particules ont une uniformité dimensionnelle de ± 15 % ou plus.

17. La composition de la revendication, dans laquelle les particules comprennent 1 % ou moins d'un polysaccharide.

18. La composition de la revendication 17, dans laquelle le polysaccharide est un alginate.

19. La composition de la revendication 18, dans laquelle l'alginate a une teneur en acide guluronique de 60 % ou plus.

20. La composition de la revendication 1, dans laquelle les particules emboliques sont insolubles dans le DMSO.

21. La composition de la revendication 1, dans laquelle les particules emboliques sont dépourvues de composés d'origine animale.

22. La composition de la revendication 1, dans laquelle l'alcool polyvinylique est composé d'un prépolymère d'alcool polyvinylique non modifié.

23. La composition de la revendication 1, dans laquelle l'alcool polyvinylique est acétalisé intrachaîne 1,3-diols.

24. La composition de la revendication 1, dans laquelle les particules sont dans un support pharmaceutiquement acceptable.

25. La composition de la revendication 24, dans laquelle le support comprend une solution saline.

26. Un procédé de fabrication de particules emboliques, comprenant :
la production de gouttes contenant un polymère de base et un composé gélifiant ;
la réaction du polymère de base ;
l'élimination du composé gélifiant ; et
la combinaison des particules avec un support pharmaceutiquement acceptable.

27. Le procédé de la revendication 26, dans lequel le composé de gélification est un polysaccharide.

28. Le procédé de la revendication 26, dans lequel le composé de gélification est un alginate.

29. Le procédé de la revendication 28, dans lequel l'alginate a une teneur en acide guluronique de 60 % ou plus.

30. Le procédé de l'une des revendications 26 à 29, comprenant la mise en contact des gouttes avec un agent gélifiant.

31. Le procédé de la revendication 30, dans lequel l'agent gélifiant est un cation divalent.

32. Le procédé de la revendication 31, dans lequel le cation est Ca⁺².

33. Le procédé de la revendication 26, dans lequel le polymère de base est le PVA.

34. Le procédé de la revendication 33, comprenant la réaction du PVA par acétalisation.

35. Le procédé de la revendication 33 ou 34, dans lequel le PVA a un poids moléculaire de 75 000 g/mole ou plus.

36. Le procédé de la revendication 26, comprenant l'abaissement de la viscosité du polymère de base et du composé de gélification lors de la formation desdites gouttes.

37. Le procédé de la revendication 36, comprenant l'abaissement de la viscosité par chauffage.

38. Le procédé de la revendication 26, comprenant la formation desdites gouttes par nébulisation vibratoire.
